# DEMANDE DE BREVET EUROPEEN

(11) **EP 4 059 544 A1**
(43) Date de publication de la demande: **21.09.2022**
(21) Numéro de dépôt: 22162715.1
(22) Date de dépôt: 17.03.2022
(51) Int. Cl.: A61M 5/158, A61M 39/02, A61M 5/32

(54) **DISPOSITIF D'INJECTION À MÉCANISME D'EXTRACTION ET PROCÉDÉ ASSOCIÉ**

(30) Priorité: 19.03.2021 FR 2102784
(71) Demandeur: Vygon, 95440 Ecouen (FR)
(72) Inventeur: WALTER, Thomas, 92500 RUEIL-MALMAISON (FR); CARPENTIER, Léa, 60000 BEAUVAIS (FR)
(74) Mandataire: Lavoix

(57) **Abrégé**

L'invention concerne un dispositif (10) d'injection comportant :
- un support d'aiguille (24) ;
- une aiguille (22) d'injection solidaire du support d'aiguille et présentant une extrémité libre (28) disposée à l'écart du support d'aiguille ; et
- un mécanisme d'extraction (50) de l'aiguille comprenant une embase (52) liée au support d'aiguille et un poussoir (54) mobile par rapport à l'embase entre une position d'utilisation de l'aiguille et une position d'extraction de l'aiguille.

L'embase (52) est déplaçable par rapport au support d'aiguille entre une position inactive du mécanisme d'extraction et une position active du mécanisme d'extraction.

Le poussoir (54) comprend une pièce externe et une pièce interne mobile par rapport à la pièce externe depuis une configuration rétractée du poussoir vers une configuration déployée du poussoir, dans laquelle la longueur du poussoir est plus grande qu'en configuration rétractée.

## Description

La présente invention concerne un dispositif d'injection comportant :
- un support d'aiguille ;
- une aiguille d'injection solidaire du support d'aiguille et présentant une extrémité libre disposée à l'écart du support d'aiguille ; et
- un mécanisme d'extraction de l'aiguille d'injection comprenant une embase liée au support d'aiguille et un poussoir mobile par rapport à l'embase entre une position d'utilisation de l'aiguille et une position d'extraction de l'aiguille, l'embase étant déplaçable par rapport au support d'aiguille entre une position inactive du mécanisme d'extraction et une position active du mécanisme d'extraction.

La présente invention concerne également un procédé de préparation d'un tel dispositif et une méthode d'injection.

Un tel dispositif est utilisé pour le traitement de certaines pathologies, pour lequel il est nécessaire d'injecter régulièrement une dose médicamenteuse liquide directement dans un organe d'un patient.

Pour ce faire, il est connu d'implanter à demeure, sur la poitrine du patient, une chambre disposée sous la peau. Cette chambre est prolongée par un tuyau cheminant dans le support jusqu'à l'organe où la dose médicamenteuse doit être distribuée. La chambre implantable comporte un réservoir présentant un opercule perforable suivant sa surface en contact avec la peau.

Pour l'injection de la dose médicamenteuse, l'aiguille du dispositif est engagée à travers la peau du patient dans la chambre implantable et la dose médicamenteuse est injectée dans cette chambre au travers de l'aiguille.

Pour extraire l'aiguille de la chambre implantable, le praticien saisit le support du dispositif et tire l'aiguille hors de la chambre.

Toutefois, la membrane de la chambre implantable étant relativement résistante, une force de traction importante doit être exercée sur l'aiguille pour permettre son extraction.

Pour éviter que le patient ne souffre du fait des forces exercées sur la peau par la chambre implantable lors du retrait de l'aiguille, il est connu d'utiliser un dispositif d'injection muni d'un mécanisme d'extraction à demeure, tel que décrit dans le document FR 2 869 806.

Un tel dispositif est muni d'un mécanisme d'extraction mécanique, monté sur le support d'aiguille du dispositif d'injection, comportant un poussoir permettant au praticien d'extraire simplement l'aiguille, de manière continue et sans risques. A cet effet, l'aiguille retenue par un support est progressivement avalée dans le poussoir lors de l'extraction.

Le mécanisme d'extraction est articulé sur le support d'aiguille, de façon à prendre une position allongée le long du corps du patient lorsque le mécanisme n'est pas utilisé, afin de réduire l'encombrement du dispositif d'extraction. Pour réduire les mouvements du mécanisme d'extraction, ce dernier est retenu en position allongée par un mécanisme d'enclenchement élastique.

Ce dispositif peut encore être amélioré. En effet, le dispositif d'extraction, même en position allongée, présente un encombrement substantiel qu'il est souhaitable de réduire. Un dispositif encombrant est plus susceptible d'être à l'origine d'erreurs de manipulation, comme par exemple des chocs accidentels sur le dispositif qui sont douloureux pour le patient à cause de l'aiguille implantée dans sa peau.

De plus, le désengagement du mécanisme d'enclenchement élastique en vue d'extraire l'aiguille peut générer des secousses désagréables lorsque l'aiguille est insérée dans le corps du patient.

Un but de l'invention est donc de fournir un dispositif d'injection moins encombrant et à la manipulation plus confortable pour le patient.

A cet effet, l'invention a pour objet un dispositif d'injection du type précité, caractérisé en ce que le poussoir comprend une pièce externe et une pièce interne mobile par rapport à la pièce externe depuis une configuration rétractée du poussoir vers une configuration déployée du poussoir, dans laquelle la longueur du poussoir est plus grande qu'en configuration rétractée.

Selon des modes de réalisation particuliers, le dispositif selon l'invention comprend l'une ou plusieurs des caractéristiques suivantes, prise(s) isolément ou selon toute combinaison techniquement réalisable :
- la pièce interne est montée coulissante dans un passage intérieur de la pièce externe ;
- le poussoir comprend un mécanisme d'enclenchement élastique, propre à bloquer la pièce interne par rapport à la pièce externe lorsque le poussoir est en configuration déployée ;
- le mécanisme d'enclenchement élastique comprend au moins une patte d'encliquetage portée par l'une de la pièce interne et de la pièce externe et au moins un logement défini par l'autre de la pièce interne et de la pièce externe, la patte d'encliquetage comportant une dent reçue dans le logement lorsque le poussoir est dans sa configuration déployée ;
- le ou chaque logement est situé proche d'une première extrémité de l'autre de la pièce interne ou de la pièce externe, de façon à recevoir une des dents lorsque le piston est en configuration déployée, le mécanisme d'enclenchement élastique comprenant en outre au moins un second logement situé proche d'une deuxième extrémité de l'autre de la pièce

interne ou de la pièce externe, de façon à recevoir la dent lorsque le piston est en configuration rétractée ;
- le mécanisme d'enclenchement élastique comprend au moins une rampe s'étendant sur l'autre de la pièce interne et de la pièce externe, la rampe délimitant un des logements à son extrémité haute, la patte d'encliquetage s'appuyant sur la rampe lors du passage du poussoir de la configuration rétractée à la configuration déployée ;
- le mécanisme d'enclenchement élastique est propre à assurer une retenue temporaire de la pièce interne par rapport à la pièce externe lorsque le poussoir est en configuration rétractée ;
- le poussoir définit, en configuration déployée, un espace interne de réception de l'aiguille ;
- le dispositif comporte un ensemble d'immobilisation temporaire de l'embase dans la position inactive, qui est actif lorsque le poussoir est en configuration rétractée et désactivé lors du passage du poussoir de la configuration rétractée à la configuration déployée ;
- l'ensemble d'immobilisation temporaire comprend une première fente s'étendant sur la pièce interne et une deuxième fente s'étendant sur la pièce externe, de largeur plus importante que la première fente, les première et deuxième fentes étant orientées vers le support d'aiguille, l'ensemble d'immobilisation temporaire comprenant de plus un barreau adapté pour s'engager dans les première et deuxième fentes, le barreau présentant une longueur supérieure à la largeur de la première fente et inférieure à la largeur de la deuxième fente ;
- la pièce interne et la pièce externe présentent des sections transversales sensiblement rectangulaires comportant avantageusement des angles arrondis ;
- l'embase est montée pivotante sur le support d'aiguille entre la position une position d'utilisation de l'aiguille et une position d'extraction de l'aiguille, autour d'un axe de pivotement ;
- l'axe de pivotement est perpendiculaire à un axe longitudinal du poussoir, en particulier à un axe de coulissement de la pièce interne par rapport à la pièce externe ;
- l'un du support d'aiguille et de l'embase comprend un téton de guidage du pivotement de l'embase par rapport au support d'aiguille, l'autre du support d'aiguille et de l'embase définit un trou d'articulation recevant le téton ;
- en position d'extraction de l'aiguille, le tronçon principal de l'aiguille s'étend totalement à l'intérieur de l'espace interne de réception défini par le poussoir ;
- l'aiguille comporte le tronçon principal du côté de son extrémité libre ;
- le tronçon principal définit l'extrémité libre de l'aiguille ;
- l'aiguille forme un coude prolongé du côté de l'extrémité libre par un tronçon principal s'étendant selon un axe d'élévation, et du côté opposé à l'extrémité libre, par un tronçon de liaison s'étendant selon un axe longitudinal du poussoir.

L'invention a également pour objet un procédé de préparation du retrait d'une aiguille préalablement introduite dans le corps d'un patient, mis en oeuvre avant le retrait de l'aiguille, comprenant les étapes suivantes :
- fourniture d'un dispositif tel que décrit plus haut, le mécanisme d'extraction occupant sa position inactive, le poussoir étant en configuration rétractée ;
- déplacement de la pièce interne par rapport à la pièce externe pour passer le poussoir de sa configuration rétractée à sa configuration déployée, augmentant la longueur du poussoir ;
- déplacement de l'embase de la position inactive à la position active du mécanisme d'extraction.

L'invention a en outre pour objet une méthode d'injection comprenant les étapes suivantes :
- fourniture d'un dispositif tel que décrit plus haut ;
- introduction de l'aiguille dans le corps du patient, la pointe perçant un opercule d'une chambre implantable ;
- injection d'une solution médicamenteuse à travers l'aiguille, dans la chambre implantable ;
- préparation du retrait de l'aiguille par le procédé tel que décrit plus haut ;
- extraction de l'aiguille en déplaçant le poussoir de la position d'utilisation de l'aiguille à la position d'extraction de l'aiguille ;

La méthode selon l'invention peut comprendre l'une ou plusieurs des caractéristiques suivantes, prise(s) isolément ou selon toute combinaison techniquement possible :
- le déplacement de la pièce interne entraine une désactivation d'un mécanisme d'immobilisation temporaire de l'embase, permettant son déplacement ;
- le tronçon principal de l'aiguille s'étend entièrement à l'intérieur du poussoir après l'extraction de l'aiguille.

L'invention sera mieux comprise à la lecture de la description qui va suivre, donnée uniquement à titre d'exemple et faite en se référant aux dessins annexés, parmi lesquels :
- la figure 1 est une vue en perspective de trois-quarts face d'un dispositif d'injection selon l'invention, dans une configuration d'utilisation de l'aiguille ;
- les figures 2 et 3 représentent des vues en coupe longitudinale, respectivement de côté et de dessus, du dispositif de la figure 1 ;
- les figures 4 et 5 représentent des vues en coupe longitudinale, respectivement de côté et de dessus, du dispositif des figures 1 à 3, le poussoir étant en configuration déployée ;
- les figures 6 et 7 représentent des vues en perspective de trois-quarts arrière et en coupe verticale respectivement, du dispositif des figures 1 à 5, le mécanisme d'extraction étant en configuration active ;
- la figure 8 est une vue en coupe verticale de côté du dispositif des figures 1 à 7, en configuration d'extraction de l'aiguille.

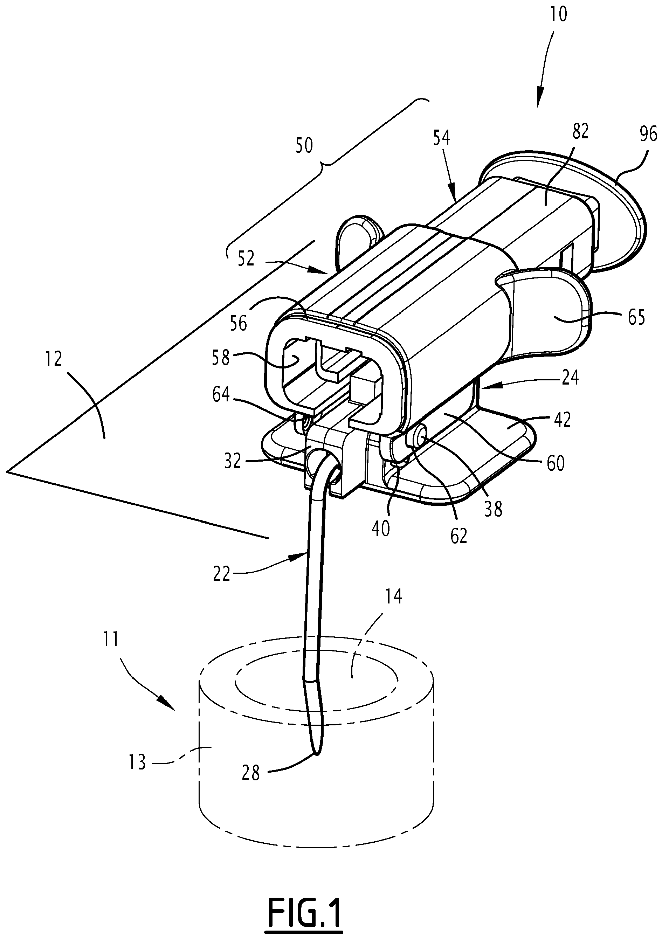

Sur les figures 1 à 8 est représenté un dispositif d'injection selon l'invention. Le dispositif est destiné à être relié à une chambre implantable (représentée schématiquement sur la figure 1) pour y injecter une solution médicamenteuse.

Comme connu en soi, la chambre implantable est disposée sous la peau d'un patient. Cette chambre présente un réservoir généralement cylindrique délimité, sur sa face en contact avec la surface intérieure de la peau, par une membrane perforable. Le réservoir est relié à un tuyau d'acheminement (non représenté) de la solution médicamenteuse vers un organe où une dose médicamenteuse doit être distribuée.

Le dispositif d'injection comporte une aiguille solidaire d'un support d'aiguille, prolongée par une tubulure reliée à l'aiguille et fixée, notamment collée au support d'aiguille. Il comporte selon l'invention un mécanisme d'extraction de l'aiguille porté par le support d'aiguille.

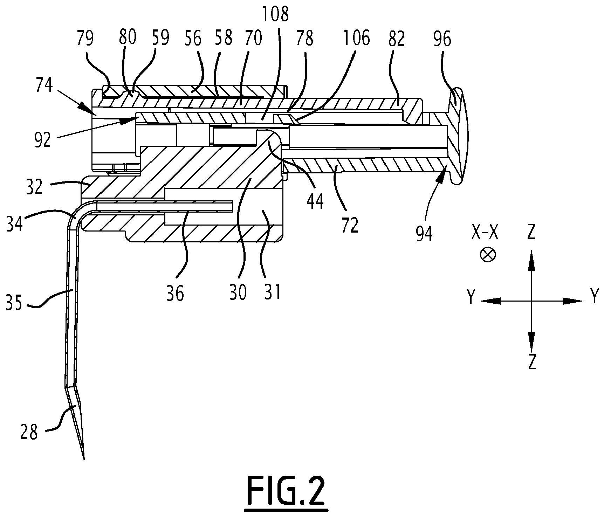

L'aiguille s'étend sensiblement selon un axe d'élévation, comme représenté sur la figure 2.

L'aiguille présente une extrémité libre en pointe courbée, de sorte que celle-ci débouche latéralement par rapport à l'axe d'élévation.

Le support comporte une âme centrale allongée selon un axe longitudinal perpendiculaire à l'axe d'élévation. L'âme centrale est sensiblement parallélépipédique. Elle est traversée de part en part par un conduit d'acheminement du liquide jusqu'à l'aiguille, s'étendant selon l'axe longitudinal. L'aiguille est solidarisée à une extrémité de l'âme centrale qui forme une tête.

L'aiguille forme un coude (voir figure 2) prolongé du côté de l'extrémité libre par un tronçon principal s'étendant selon l'axe d'élévation, et du côté opposé à l'extrémité libre, par un tronçon de liaison s'étendant selon l'axe longitudinal. Le tronçon de liaison est engagé dans le conduit axial traversant l'âme centrale, depuis la tête.

Le support comprend deux tétons d'articulation, visibles sur la figure 1, qui font saillie latéralement de part et d'autre de l'âme centrale, à proximité de la tête. Les tétons d'articulation s'étendent selon un axe transversal perpendiculaire à l'axe d'élévation et à l'axe longitudinal.

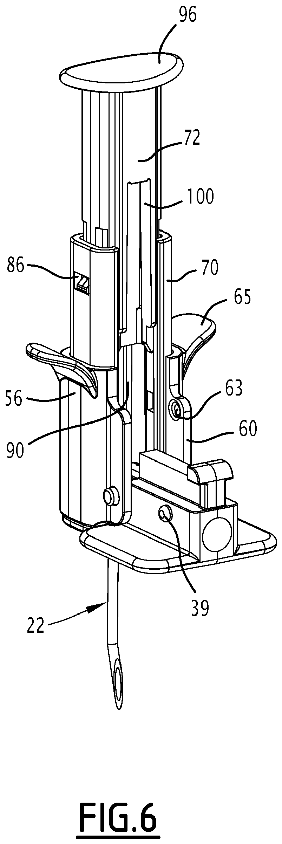

Le support comprend de plus deux tétons de retenue basse (dont l'un est visible figure 6) et deux tétons de retenue haute (dont l'un est visible figure 1), qui font saillie latéralement depuis l'âme centrale, parallèlement à l'axe transversal.

Les tétons de retenue basse s'étendent au même niveau que les tétons d'articulation selon l'axe d'élévation, depuis une partie de l'âme centrale plus éloignée de la tête selon l'axe longitudinal.

Les tétons de retenue basse s'étendent en dessous des tétons d'articulation selon l'axe d'élévation.

Les tétons de retenue basse et les tétons de retenue haute présentent une étendue transverse moins importante que les tétons d'articulation, selon l'axe transversal.

Du côté du support tourné vers l'aiguille, le support comporte une platine d'appui s'étendant suivant la longueur du support. La tête fait saillie par rapport à cette platine d'appui selon l'axe longitudinal.

Du côté du support opposé à l'aiguille, le support comporte un barreau de blocage du mécanisme d'extraction. Le barreau s'étend au-dessus de l'âme centrale, du côté opposé à la tête selon l'axe longitudinal. Il fait saillie latéralement depuis les deux côtés de l'âme centrale selon une direction parallèle à l'axe transversal.

Le dispositif d'injection comporte selon l'invention un mécanisme d'extraction de l'aiguille qui est lié à demeure au support d'aiguille en étant déplaçable par rapport à celui-ci entre une position inactive du mécanisme d'extraction telle qu'illustrée aux figures 1 à 5 et une position active du mécanisme d'extraction telle qu'illustrée sur les figures 6 à 8.

Le mécanisme d'extraction comporte une embase et un poussoir monté coulissant par rapport à l'embase. L'embase est déplaçable par rapport au support et est notamment articulée par rapport à celui-ci autour des tétons d'articulation.

L'embase comprend un manchon délimitant un passage de circulation du poussoir.

Lorsque le mécanisme d'extraction est en position inactive, le manchon s'étend sensiblement selon une direction parallèle à l'axe longitudinal.

Lorsque le mécanisme d'extraction est basculé en position active, le manchon s'étend sensiblement selon une direction parallèle à l'axe d'élévation.

Le manchon est ouvert latéralement sur une largeur correspondant à la largeur de l'âme centrale du support. Le manchon définit de plus dans sa face supérieure une cavité de retenue débouchant dans le passage interne.

L'embase comprend également deux flancs prolongeant le manchon de part et d'autre de cette ouverture, propres à s'engager autour de l'âme centrale. Pour l'articulation de l'embase sur l'âme centrale, les flancs présentent deux trous d'articulation dans lesquels sont reçus les tétons d'articulation.

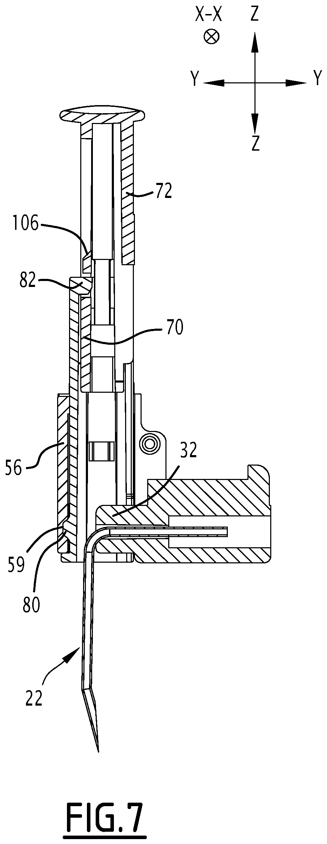

L'axe d'articulation est parallèle à l'axe transversal, au voisinage de la tête de sorte qu'en position basculée, la tête s'étend à l'intérieur du passage, comme représenté figure 7.

Les flancs présentent de plus deux orifices de retenue basse, visibles sur la figure 7, et deux orifices de retenue haute, visibles sur la figure 1, s'ouvrant dans des faces internes des flancs.

Les orifices de retenue basse sont adaptés pour recevoir les tétons de retenue basse lorsque le mécanisme d'extraction est en position inactive, et ainsi permettre une retenue temporaire du mécanisme d'extraction en position inactive.

De même, les orifices de retenue haute sont adaptés pour recevoir les tétons de retenue haute lorsque le mécanisme d'extraction est en position active, et ainsi permettre une retenue temporaire du mécanisme d'extraction en position active.

L'embase comporte en outre deux appui-doigt latéraux, faisant saillie depuis le manchon à l'extrémité opposée à la tête.

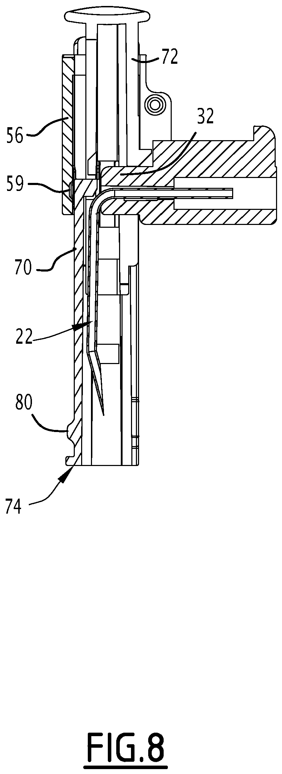

Le poussoir est reçu coulissant à l'intérieur du passage entre une position d'utilisation de l'aiguille telle qu'illustrée sur les figures 1 à 7 et une position d'extraction de l'aiguille telle qu'illustrée sur la figure 8.

Le poussoir comprend une pièce externe montée coulissante dans l'embase, et une pièce interne montée coulissante dans la pièce externe de manière télescopique avec la pièce externe. Le poussoir comprend de plus un mécanisme d'enclenchement élastique.

La pièce externe est une chemise évidée présentant une section complémentaire de celle du passage interne. Cette section est ici sensiblement rectangulaire aux angles arrondis.

La pièce externe est ouverte à une extrémité avant, située proche de la tête, et à une extrémité arrière, située plus loin de la tête. La pièce externe définit un passage intérieur débouchant à l'extrémité avant et à l'extrémité arrière.

La pièce externe définit un épaulement externe sur le tour de son extrémité avant, et un plot de retenue dépassant d'une face supérieure, proche de l'extrémité avant. Le plot de retenue est propre à s'insérer dans la cavité dans la position d'utilisation de l'aiguille. L'épaulement externe est propre à s'appuyer contre le bord avant du manchon lorsque le plot de retenue est reçu dans la cavité.

La pièce externe définit une fente externe orientée vers le support d'aiguille, ouverte et de l'extrémité avant à l'extrémité arrière et débouchant sur le passage intérieur. La fente externe présente une largeur supérieure à la longueur du barreau.

La pièce interne est une chemise évidée présentant une section complémentaire à celle du passage intérieur. Cette section est ici sensiblement rectangulaire aux angles arrondis.

La pièce interne présente une extrémité avant ouverte et une extrémité arrière 94 munie d'un appui-doigt arrière. La pièce interne définit un espace interne, débouchant à l'extrémité avant.

La pièce interne définit de plus une fente interne, orientée vers le support d'aiguille. La fente interne est ouverte de l'extrémité avant à l'extrémité arrière, et débouche sur l'espace interne. La fente interne présente une largeur inférieure à la longueur du barreau.

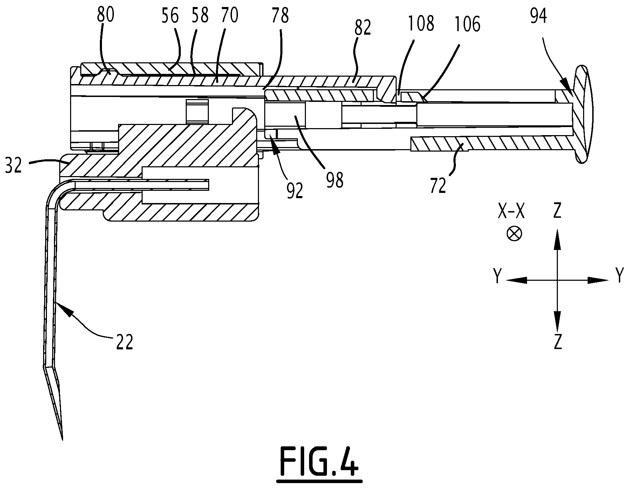

La pièce interne est montée coulissante dans le passage interne de la pièce externe, le long d'un axe longitudinal du poussoir. La pièce interne est ainsi mobile depuis une configuration rétractée du poussoir, représentée sur les figures 1 à 3, et une configuration déployée du poussoir, représentée sur les figures 4 à 8, dans laquelle la longueur du poussoir est plus grande qu'en configuration rétractée.

Lorsque le poussoir est en configuration déployée, le passage interne de la pièce externe et l'espace intérieur de la pièce externe constituent un espace interne continu de réception de l'aiguille défini par le poussoir, comme représenté sur la figure 8.

Le barreau, la fente externe et la fente interne forment un ensemble d'immobilisation temporaire de l'embase dans la position inactive.

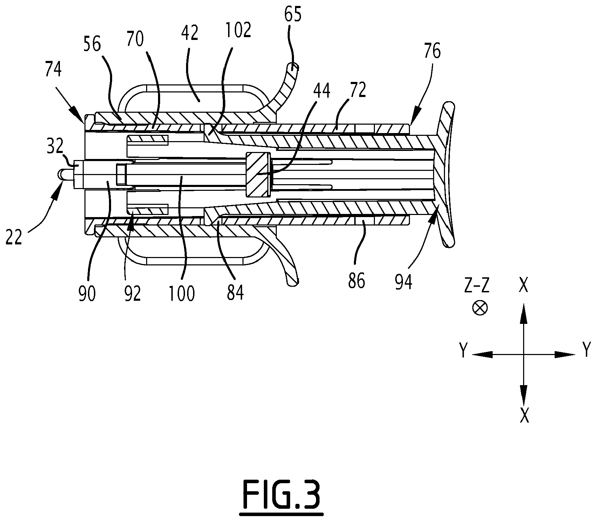

L'ensemble d'immobilisation temporaire est actif lorsque le poussoir est en configuration rétractée, comme représenté sur la figure 3. Le barreau s'étend dans l'espace interne, engagé à travers la fente externe et la fente interne. La longueur du barreau étant supérieure à la largeur de la fente interne, le barreau bloque le déplacement du poussoir à l'écart du support. L'embase est ainsi immobilisée en position inactive du mécanisme d'extraction.

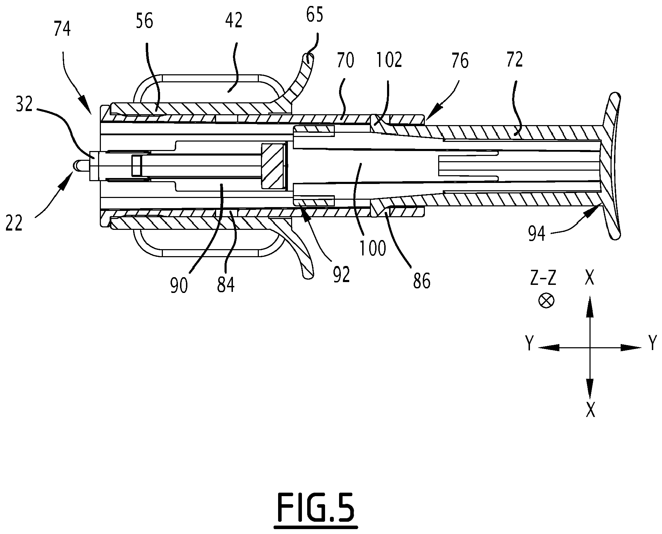

L'ensemble d'immobilisation temporaire est désactivé lors du passage du poussoir de la configuration rétractée à la configuration déployée, comme représenté sur la figure 5. Dans cette configuration, la pièce interne s'est déplacée le long de l'axe longitudinal à l'écart du barreau. Le barreau n'est alors plus engagé que dans la fente externe, dont la largeur est plus grande que la longueur du barreau. L'embase peut alors être déplacée vers la position active du mécanisme d'extraction.

Le mécanisme d'enclenchement élastique est propre à bloquer la pièce interne dans son mouvement par rapport à la pièce externe lorsque le poussoir est en configuration déployée.

Le mécanisme d'enclenchement élastique comprend une patte d'encliquetage supérieure portée par la pièce externe sur une face supérieure, au niveau de l'extrémité arrière. La patte d'encliquetage supérieure porte une dent à son extrémité libre, orientée vers le passage intérieur.

Le mécanisme d'enclenchement élastique comprend de plus deux pattes latérales d'encliquetage portées par la pièce interne le long de deux faces latérales, chaque patte latérale d'encliquetage portant une dent à une extrémité libre, orientée vers l'extérieur. Il comprend des logements avant et des logements arrière définis dans des faces latérales de la pièce externe, représentés sur les figures 3 et 5.

Les dents des pattes latérales d'encliquetage sont asymétriques, c'est-à-dire qu'elles présentent un côté bloquant et un côté passant. Par exemple, la normale au côté bloquant forme un angle sensiblement nul avec la patte d'encliquetage latérale, alors que la normale au côté passant forme un angle compris entre 30° et 60° avec la patte d'encliquetage latérale.

Le mécanisme d'enclenchement élastique comporte également une rampe s'étendant sur une face supérieure de la pièce interne, ainsi qu'un logement supérieur, la rampe délimitant le logement supérieur à son extrémité haute.

En configuration déployée, chacune des dents portées par les pattes latérales d'encliquetage est reçue par un des logements arrière, comme représenté sur la figure 5, et la dent portée par la patte d'encliquetage supérieure est reçue par le logement supérieur, comme représenté sur la figure 4.

Avantageusement, le mécanisme d'enclenchement élastique est propre à assurer une retenue temporaire de la pièce interne par rapport à la pièce externe lorsque le poussoir est en configuration rétractée.

Par retenue temporaire, on entend que l'effort nécessaire pour que le poussoir quitte la configuration rétractée est suffisamment important pour que le poussoir ne quitte pas la configuration déployée sans manipulation par un utilisateur, mais suffisamment faible pour qu'une telle manipulation par l'utilisateur ne provoque pas d'à-coups dans le dispositif d'injection lorsque le poussoir quitte la configuration rétractée. Cet effort est avantageusement compris entre 6 N et 10 N.

En configuration rétractée, chacune des dents portées par les pattes latérales d'encliquetage est reçue par un des logements avant définis par la pièce externe. Lorsque le poussoir est déplacé de la configuration rétractée vers la configuration déployée, le côté passant de chacune des dents s'appuie sur un bord du logement avant, permettant à la dent de sortir du logement avant lorsque l'utilisateur tire sur la pièce interne, avec un effort nécessaire réduit.

Inversement, lorsque le poussoir est en configuration déployée, chacune des dents portées par les pattes latérales d'encliquetage est reçue par un des logements arrière 86. Le côté bloquant de chacune des dents s'appuie sur un bord du logement arrière, empêchant la dent de sortir du logement arrière. Le mécanisme d'enclenchement élastique s'oppose alors au déplacement de la pièce interne à l'écart de la configuration déployée.

Le procédé d'utilisation du dispositif d'injection, et plus spécifiquement de retrait de l'aiguille, va maintenant être décrit.

Le dispositif d'injection est initialement dans la configuration représentée sur les figures 1 à 3, avec l'aiguille traversant la peau d'un patient pour atteindre une chambre implantable située en dessous. La platine d'appui est en appui sur la peau du patient.

Le poussoir occupe sa configuration rétractée et le mécanisme d'extraction se trouve en position inactive. Le poussoir s'étend sensiblement parallèle à l'axe longitudinal.

Le piston est en positon d'utilisation de l'aiguille, avec l'épaulement de l'extrémité avant de la pièce externe en appui contre l'avant du manchon, proche de la tête. Le plot de retenue est engagé dans la cavité de retenue, de manière à maintenir le poussoir en position d'utilisation de l'aiguille.

Les dents des pattes latérales d'encliquetage sont engagées dans les logements avant, exerçant une retenue du poussoir en configuration rétractée.

Le barreau est situé dans l'espace interne de la pièce interne, engagé à la fois dans la fente interne et dans la fente externe, qui sont superposées. Comme la longueur du barreau est supérieure à la largeur de la fente interne, le barreau bloque complètement le piston contre l'âme centrale et empêche le basculement de l'embase vers la position active.

La longueur de poussoir, en configuration rétractée, est minimisée, diminuant l'encombrement du dispositif sur le patient.

Lors d'une première étape, l'utilisateur exerce une traction sur la pièce interne par l'appui-doigt arrière, de façon à déplacer le poussoir de sa configuration rétractée à sa configuration déployée, représentée sur les figures 4 à 8.

La partie interne coulisse dans la partie externe le long de l'axe. La longueur du poussoir augmente, pour présenter, en configuration déployée, une longueur supérieure à 120%, avantageusement supérieure à 125% de la longueur dans la configuration rétractée.

Les dents des pattes d'encliquetage latérales sortent des logements avant, puis s'engagent dans les logements arrière en configuration déployée. La dent de la patte d'encliquetage parcourt la rampe et s'engage dans le logement supérieur en configuration déployée. Le mécanisme d'enclenchement élastique bloque alors le poussoir en configuration déployée.

Le barreau est dégagé de la fente interne et n'est plus engagé que dans la fente externe. Il ne bloque alors plus le poussoir contre l'âme centrale. Les plots de retenue basse sont engagés dans les orifices de retenue basse, et exercent une retenue de l'embase en position inactive.

Lors d'une deuxième étape, l'utilisateur bascule l'embase autour de l'axe d'articulation, de la position inactive vers la position active. Dans la position active, représentée sur les figures 6 à 8, le piston s'étend sensiblement parallèle à l'axe d'élévation.

La tête est alors reçue dans le passage intérieur. Les tétons de retenue haute sont engagés dans les orifices de retenue haute, de façon à exercer une retenue de l'embase en position active.

Lors d'une troisième étape, l'utilisateur exerce une poussée sur l'appui-doigt arrière avec le pouce en retenant les appui-doigts latéraux avec deux autres doigts, de façon à faire coulisser le piston dans le passage. Le piston est alors déplacé d'une position d'utilisation de l'aiguille, représentée sur les figures 6 et 7, à une position d'extraction de l'aiguille, représentée sur la figure 8.

Lors du passage de la position d'utilisation de l'aiguille à la position d'extraction de l'aiguille, le plot de retenue sort de la cavité de retenue sous l'effet de la poussée exercée par l'utilisateur.

L'épaulement de l'extrémité avant de la pièce externe prend alors appui sur la peau du patient, permettant d'avoir une surface d'appui suffisante, et la poussée de l'utilisateur sur le piston fait remonter l'embase le long du piston, extrayant l'aiguille du corps du patient. Le piston avale progressivement l'aiguille.

En position d'extraction de l'aiguille, le tronçon principal de l'aiguille s'étend totalement à l'intérieur de l'espace de réception du poussoir, l'extrémité avant étant située au-delà de l'extrémité libre. Ceci permet de protéger l'utilisateur en prévenant tout risque de piqûre accidentelle.

On conçoit qu'avec un tel dispositif d'injection, l'encombrement du mécanisme d'extraction est plus faible, notamment lorsque le poussoir est en position rétractée et présente une longueur réduite. Ceci permet de réduire l'espace occupé par le dispositif d'injection sur la peau du patient, réduisant ainsi les risques d'erreurs de manipulation lors de l'injection de la solution médicamenteuse, ainsi que les risques de basculement causés par l'effet balancier du piston. L'encombrement réduit du dispositif d'injection améliore également le confort du patient.

De plus, le dispositif comprend un mécanisme d'enclenchement élastique formé de plusieurs pattes d'encliquetage et les logements associés, ce qui assure un verrouillage sûr du poussoir en configuration déployée, afin de garantir une extraction efficace.

Enfin, l'ensemble d'immobilisation temporaire de l'embase en position inactive, combiné à la retenue temporaire du piston en configuration rétractée assurée par le mécanisme d'enclenchement élastique, permet d'assurer que le l'embase et le piston restent bien en position inactive sans manipulation extérieure d'un opérateur. Ceci réduit encore les risques d'erreurs de manipulation.

## Revendications

1. Dispositif (10) d'injection comportant :
- un support d'aiguille (24) ;
- une aiguille (22) d'injection solidaire du support d'aiguille (24) et présentant une extrémité libre (28) disposée à l'écart du support d'aiguille (24) ; et
- un mécanisme d'extraction (50) de l'aiguille (22) d'injection comprenant une embase (52) liée au support d'aiguille (24) et un poussoir (54) mobile par rapport à l'embase (52) entre une position d'utilisation de l'aiguille (22) et une position d'extraction de l'aiguille (22), l'embase (52) étant déplaçable par rapport au support d'aiguille (24) entre une position inactive du mécanisme d'extraction (50) et une position active du mécanisme d'extraction (50),
**caractérisé en ce que** le poussoir (54) comprend une pièce externe (70) et une pièce interne (72) mobile par rapport à la pièce externe (70) depuis une configuration rétractée du poussoir (54) vers une configuration déployée du poussoir (54), dans laquelle la longueur du poussoir (54) est plus grande qu'en configuration rétractée.

2. Dispositif (10) selon la revendication 1, dans lequel la pièce interne (72) est montée coulissante dans un passage intérieur (78) de la pièce externe (70).

3. Dispositif (10) selon l'une quelconque des revendications 1 ou 2, dans lequel le poussoir (54) comprend un mécanisme d'enclenchement élastique, propre à bloquer la pièce interne (72) par rapport à la pièce externe (70) lorsque le poussoir (54) est en configuration déployée.

4. Dispositif (10) selon la revendication 3, dans lequel le mécanisme d'enclenchement élastique comprend au moins une patte d'encliquetage (82, 102) portée par l'une de la pièce interne (72) et de la pièce externe (70) et au moins un logement (86, 108) défini par l'autre de la pièce interne (72) et de la pièce externe (70), la patte d'encliquetage (82, 102) comportant une dent reçue dans le logement (86, 108) lorsque le poussoir (54) est dans sa configuration déployée.

5. Dispositif (10) selon la revendication 4, dans lequel le ou chaque logement (86, 108) est situé proche d'une première extrémité (76) de l'autre de la pièce interne (72) ou de la pièce externe (70), de façon à recevoir une des dents lorsque le piston (54) est en configuration déployée, le mécanisme d'enclenchement élastique comprenant en outre au moins un second logement (84) situé proche d'une deuxième extrémité (74) de l'autre de la pièce interne (72) ou de la pièce externe (70), de façon à recevoir la dent lorsque le piston (54) est en configuration rétractée.

6. Dispositif (10) selon l'une quelconque des revendications 4 ou 5, dans lequel le mécanisme d'enclenchement élastique comprend au moins une rampe (106) s'étendant sur l'autre de la pièce interne (72) et de la pièce externe (70), la rampe (106) délimitant un des logements (108) à son extrémité haute, la patte d'encliquetage (82) s'appuyant sur la rampe (106) lors du passage du poussoir (54) de la configuration rétractée à la configuration déployée.

7. Dispositif (10) selon l'une quelconque des revendications 3 à 6, dans lequel le mécanisme d'enclenchement élastique est propre à assurer une retenue temporaire de la pièce interne (72) par rapport à la pièce externe (70) lorsque le poussoir (54) est en configuration rétractée.

8. Dispositif (10) selon l'une quelconque des revendications 1 à 7, dans lequel le poussoir (54) définit, en configuration déployée, un espace interne de réception de l'aiguille (22).

9. Dispositif (10) selon l'une quelconque des revendications 1 à 8, dans lequel le dispositif (10) comporte un ensemble d'immobilisation temporaire de l'embase (52) dans la position inactive, qui est actif lorsque le poussoir (54) est en configuration rétractée et désactivé lors du passage du poussoir (54) de la configuration rétractée à la configuration déployée.

10. Dispositif (10) selon la revendication 9, dans lequel l'ensemble d'immobilisation temporaire comprend une première fente (100) s'étendant sur la pièce interne (72) et une deuxième fente (90) s'étendant sur la pièce externe (70), de largeur plus importante que la première fente (100), les première (100) et deuxième fentes (90) étant orientées vers le support d'aiguille, l'ensemble d'immobilisation temporaire comprenant de plus un barreau (44) adapté pour s'engager dans les première (100) et deuxième (90) fentes, le barreau (44) présentant une longueur supérieure à la largeur de la première fente (100) et inférieure à la largeur de la deuxième fente (90).

11. Dispositif (10) selon l'une quelconque des revendications 1 à 10, dans lequel la pièce interne (72) et la pièce externe (70) présentent des sections transversales sensiblement rectangulaires comportant des angles arrondis.

12. Procédé de préparation du retrait d'une aiguille (22) préalablement introduite dans le corps d'un patient, mis en œuvre avant le retrait de l'aiguille (22), comprenant les étapes suivantes :
- fourniture d'un dispositif (10) selon l'une quelconque des revendications 1 à 11, le mécanisme d'extraction (50) occupant sa position inactive, le poussoir (54) étant en configuration rétractée ;
- déplacement de la pièce interne (72) par rapport à la pièce externe (70) pour passer le poussoir (54) de sa configuration rétractée à sa configuration déployée, augmentant la longueur du poussoir (54) ;
- déplacement de l'embase (52) de la position inactive à la position active du mécanisme d'extraction (50).
